# FASCICULE DE BREVET EUROPEEN

(11) **EP 0 494 296 B1**
(45) Date de publication et mention de la délivrance du brevet: **12.06.2002**
(21) Numéro de dépôt: 91914204.2
(22) Date de dépôt: 25.07.1991
(51) Int. Cl.: C12N 15/29, C12N 15/82, A01H 5/00

(54) **NOUVEAU COMPLEXE GENIQUE UTILE NOTAMMENT POUR LA MODIFICATION DES VEGETAUX TELS QUE CUCUMIS MELO ET CUCUMIS MELO INCORPORANT CE COMPLEXE GENIQUE**
INSBESONDERE ZUR MODIFIZIERUNG VON PFLANZEN WIE CUCUMIS MELO VERWENDBARER GENKOMPLEX UND DIESEN ENTHALTENDE CUCUMIS MELO
NOVEL GENE COMPLEX PARTICULARLY USEFUL FOR MODIFYING PLANTS SUCH AS CUCUMIS MELO, AND CUCUMIS MELO INCORPORATING SAID GENE COMPLEX

(30) Priorité: 27.07.1990 FR 9009622
(43) Date de publication de la demande: 15.07.1992
(73) Titulaire: BIO-OBTENTION SC, 34980 Montferrier-sur-Lez (FR)
(72) Inventeur: DREYER, Alain, F-84470 Châteauneuf-de-Gadagne (FR); GINOUX, Jean-Paul, F-13630 Eyragues (FR)
(74) Mandataire: Warcoin, Jacques
(86) Numéro de dépôt international: FR9100618
(87) Numéro de publication internationale: WO9202622

(56) Documents cités:
- WO-A-91/01375
- WO-A-91/09112
- NATURE, Vol. 346, 19 July 1990, HAMILTON A.J. et al., "Antisense Gene That Inhibits Synthesis of the Hormone Ethylene in Transgenic Plants", pages 284-287.
- J. SCI. FOOD AGRIC., Vol. 31, No. 6, 1980, HOBSON G.E. et al., "Effect of the Introduction of Non-ripening Mutant Genes on the Composition and Enzyme Content of Tomato Fruit", pages 578-584.
- PLANT PHYSIO, Vol. 52, No. 1, 1973, HERNER R.C. et al., "Ethylene Production and Respiratory Behaviour of the Rin Tomato Mutant", pages 38-42.
- HORTSCIENCE, Vol. 23, No. 4, 1988, KENDALL S.A. et al., "Genetic Variation of Ethylene Production in Harvested Muskmelon Frutis", pages 759-761.
- HORTSCIENCE, Vol. 16, No. 3, 1981, Sect. 2, KENDALL S.A. et al., "A Diallel Analysis of Fruit Maturation and Ripening in Muskmelon Cucumis-melo", page 461.
- TIBTECH, Vol. 7, No. 7, July 1990, KRAMER M. et al., "Progress Towards the Genetic Engineering of Tomato Fruit Softening", pages 191-194.

## Description

La présente invention concerne un nouveau complexe génique utile notamment pour la modification des végétaux, notamment des *Cucumis melo*.

Elle concerne également le *Cucumis melo* et les cellules végétales modifiés par ledit complexe génique.

Dans le domaine des fruits, l'étape clé pour les produits qui doivent être commercialisés est celle de la maturation. La maturation est une séquence d'événements qui commence généralement à la fin de la croissance du fruit et qui doit aboutir à des qualités d'ensemble optimales pour la consommation, notamment pour ce qui est des qualités organoleptiques.

Si le fruit dépasse cette maturité, il se produit une désorganisation cellulaire qui correspond à ce qu'on appelle la surmaturation Cette surmaturation correspond pour certains fruits à la fin d'un paroxysme de production d'éthylène.

C'est pourquoi les fruits sont répartis en deux groupes qui tiennent compte de leur comportement avec l'éthylène :
* Les fruits climatériques qui dégagent de l'éthylène de façon autocatalytique au début de la phase de maturation.
   Chez ces fruits, l'éthylène semble initier, coordonner et accélérer l'ensemble des processus physiologiques de maturation.
* Les fruits non climatériques, qui ne présentent pas cette crise éthylénique autocatalytique au début de leur phase de mûrissement, et chez lesquels il n'est pas possible de modifier la maturation par application d'éthylène.

Les fruits non climatériques sont notamment les agrumes, les raisins et les fraises.

Les fruits climatériques sont notamment les pommes, les poires, les tomates, les melons et les bananes.

Les fruits climatériques présentent la propriété sur le plan de la conservation de passer très rapidement de l'état mature à l'état surmature après l'apparition du paroxysme de la crise éthylénique.

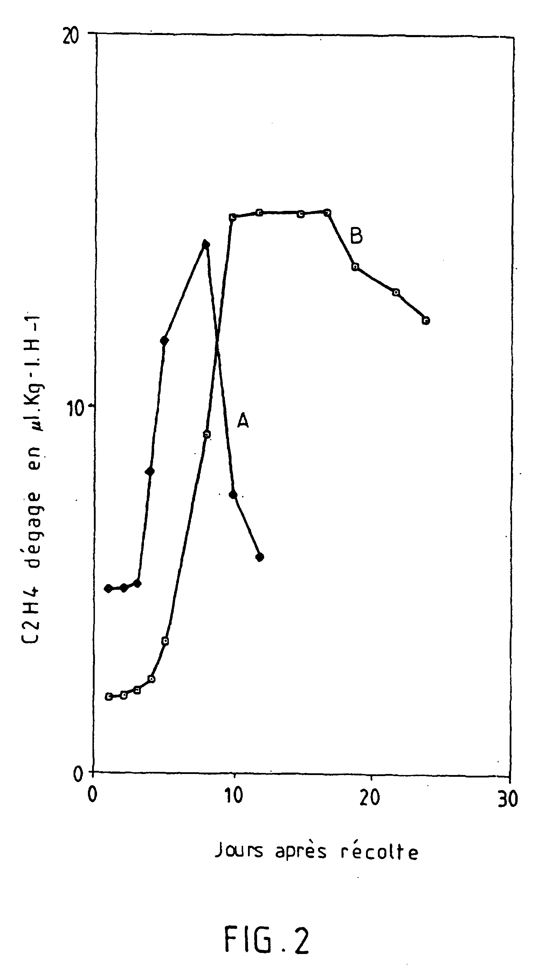

Comme cela peut être observé dans la figure 2 (courbe A) relative à *Cucumis melo*, la crise éthylénique est très importante et peu de temps après le fruit commence à se désorganiser et sa valeur marchande diminue.

La demande internationale WO 91/01375 concerne des plants de tomates transgéniques modifiés de manière à réduire la production d'éthylène.

L'article paru dans *J*. *Sci*. *Food Agric*., 1980, **31**(6) 578-84, décrit l'introduction d'un gène de non mûrissement dans une variété de tomate Ailsa Craig et les auteurs ont montré que cette variété produit des tomates à haute teneur en acide et de basse teneur en sucre qui mûrissent lentement.

L'article paru dans *Nature,* **346,** 19 juillet 1990, décrit un gène antisens introduit dans le plasmide pTOM13, qui produit un polypeptide impliqué dans la conversion du 1-aminocyclopropane-1-acide carboxylique en éthylène par l'enzyme ACC-oxydase.

Ce gène antisens pTOM13 est transféré par l'intermédiaire de BIN19 dans Agrobactérium tumefaciens qui est utilisé pour transformer les plants de tomates. Ce RNA antisense est d'ailleurs lui-même obtenu à partir de feuilles de tomates.

L'article paru dans *Hort*. *Science,* 1988, **23,** n°4, pages 759-761, rassemble les résultats obtenus en étudiant la concentration d'éthylène et la vitesse de production d'éthylène des fruits mûrs, en particulier dans des melons brodés ou non, ainsi que dans leurs hybrides; les auteurs montrent que la production d'éthylène dans le melon est sous contrôle génétique, et arrivent à la conclusion que l'on pourrait améliorer la durée de stockage des melons en sélectionnant des génotypes permettant une fabrication faible d'éthylène.

La présente invention concerne donc la mise en évidence d'un complexe génique obtenu à partir d'un système cellulaire végétal, caractérisé en ce que, lorsqu'on le transfère dans un système cellulaire compatible appartenant à une plante portant des fruits de type climatérique, il s'exprime en conférant aux fruits des caractéristiques de type non climatérique.

Ce complexe génique est plus particulièrement intéressant lorsqu'il s'exprime dans Cucumis melo.

Toutefois, il est bien évident que ce complexe génique peut également être utilisé pour transformer d'autres fruits climatériques en fruits non climatériques, ceci en utilisant plus particulièrement les techniques de génie génétique développées dans le domaine végétal .

De façon encore plus spécifique, la présente invention concerne un complexe génique obtenu à partir d'un système cellulaire végétal mutant du type Cucumis melo, caractérisé en ce que, lorsqu'il est introduit dans un Cucumis melo, il fait s'exprimer dans ledit système cellulaire les phénotypes suivants, détectables au cours du temps et définis par comparaison avec ledit système cellulaire non modifié:
a) une émission d'éthylène présentant un plateau stable pendant environ 5 jours,
b) une diminution de la teneur en fructose,
c) une stabilité-de la teneur en glucose dans le temps,
d) une diminution de la teneur en acide malique,
e) une augmentation sensible de la teneur en acide propionique dès l'introduction du complexe génique dans ledit système cellulaire, suivie d'une inversion de son processus d'évolution.

Elle possède également les caractéristiques suivantes:
f) une augmentation importante de la teneur en protéine totale,
g) une augmentation sensible de la teneur en acide gras (sous forme Methyl Ester) avec notamment l'apparition d'acide gras à chaine courte "(en dessous de C12)"
h) une augmentation de la résistance de la texture de la chair qui devient beaucoup plus résistante au vieillissement (notamment à partir de la maturité).

La mise en évidence et la mesure de ces différents paramètres seront explicitées plus en détail dans les exemples.

Ce complexe génique est d'origine naturelle, il est stable et autoreproductible dans les systèmes cellulaires, notamment les systèmes cellulaires du type Cucumis melo.

Actuellement, il peut être reproduit en suivant le même processus mais à l'aide de systèmes qui permettent rapidement de tester les produits les plus prometteurs. En particulier, ce complexe peut être obtenu à partir de systèmes cellulaires de type climatérique en utilisant des procédés de mutation physiques ou chimiques et en sélectionnant les produits obtenus par leur teneur en acide propionique.

En effet, le complexe selon la présente invention confère aux produits qui l'incorporent la propriété de présenter une teneur forte en acide propionique qui peut être mise en évidence par divers moyens, notamment une mesure HPLC. Les exemples donneront d'autres informations sur ce type de procédé.

Lorsque l'on a pu mettre en évidence un système cellulaire exprimant ce complexe génique, il suffit de reproduire le système cellulaire porteur du complexe génique objet du brevet avec n'importe quel système cellulaire compatible ou rendu compatible. Ceci permet d'obtenir un nouveau système cellulaire dans lequel le complexe génique s'exprime.

En outre, le complexe génique selon la présente invention a été introduit dans la lignée 95LS444 de Cucumis melo dont les semences ont été déposées conformément au Traité de Budapest dans la Collection NCIMB (National Collection of Industrial and Marine Bacteria - ABERDEEN AB2 1 RY (Ecosse - GB) 23 St. Machar Drive) le 19 juillet 1990 sous le n° 40310.

A partir de ces semences, l'homme de métier peut aisément reproduire l'invention en utilisant des techniques connues.

Si l'on reproduit le système cellulaire porteur du complexe génique selon l'invention avec n'importe quel système cellulaire compatible ou rendu compatible, on obtient un système cellulaire exprimant le complexe génique.

Les descendances de ce croisement donnent une ségrégation dans lequel il est toujours possible de récupérer le complexe génique en utilisant comme crible les analyses biochimiques caractéristiques décrites précédemment et ainsi de suite.

On peut ainsi réobtenir de nouveaux systèmes cellulaires stables et auto-reproductibles dans lesquels le complexe génique de l'invention s'exprime.

En outre, il est possible, comme cela a été réalisé à de nombreuses reprises dans les végétaux, de transférer ce complexe génique dans d'autres systèmes cellulaires, notamment par des techniques de fusion cellulaire et de fusion de protoplaste, comme cela a été réalisé déjà, par exemple, pour l'obtention de stérilités mâles cytoplasmiques, lesquelles ont été transférées du choux au colza.

Les techniques de fusion cellulaire ou de protoplaste ne seront pas rappelées ici en détail, elles sont connues de l'homme de métier.

C'est pourquoi, la présente invention concerne également les complexes géniques obtenus à partir de la lignée 95LS444 de Cucumis melo déposée à la Collection NCIMB sous le n" 40310.

La présente invention concerne également les cellules, notamment les cellules végétales, modifiées par introduction dudit complexe génique.

La présente invention concerne également un Cucumis melo modifié par un complexe génique obtenu à partir d'un système cellulaire végétal mutant du type Cucumis melo faisant s'exprimer dans ledit Cucumis melo modifié les phénotypes suivants, détectables au cours du temps et définis par comparaison avec le système cellulaire non modifié :
a) une émission d'éthylène présentant un plateau stable pendant environ 5 jours,
b) une diminution de la teneur en fructose,
c) une stabilité de la teneur en glucose dans le temps,
d) une diminution de la teneur en acide malique,
e) une augmentation sensible de la teneur en acide propionique dès l'introduction du complexe génique dans ledit système cellulaire, suivie d'une inversion de son processus d'évolution.

La présente invention concerne également un Cucumis melo tel que décrit ci-dessus, caractérisé en ce qu'en outre :
- il présente un dégagement de C₂H₄ de l'ordre d'au moins 10 µl kg⁻¹.h⁻¹, après 7 jours, de préférence 12 jours après le maximum de dégagement de C₂H₄,
- sa teneur en acide propionique diminue du premier au septième jour.
- sa teneur en azote total par rapport à la matière sèche est supérieure à 1,8 %,
- le pourcentage en Acide γ-aminobutyrique (GABA) par rapport aux acides aminés totaux (ASP, GLU, ASN, SER, HIS, GLY, THR, ARG, ALA, TYR, GABA, MET, VAL, PHE, ILEU, LEU, ORN, LYS, est supérieur à 6 %,
- le pourcentage en C16-1 Acide palmitoléîque par rapport aux acides gras totaux est supérieur à 6 %,
- le pourcentage en C18-1 Acide oléïque par rapport au acides gras totaux est inférieur à 10 %.
- Il contient des acides gras en dessous de "C12".

Le melon selon la présente invention comporte également un taux de fructose croissant beaucoup, notamment doublant du premier au septième jour suivant la maturation.

Les exemples ci-après vont permettre de mettre en évidence d'autres caractéristiques et avantages de la présente invention.

La figure 1 représente l'intensité respiratoire de C. melo témoin 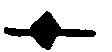 et C. melo modifié selon l'invention 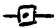 en fonction du nombre de jours après la récolte.

La figure 2 représente la quantité de C₂H₄ dégagée pour le témoin 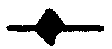 et le produit selon l'invention 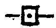 en fonction du nombre de jours après la récolte.

La figure 3 représente le pourcentage de fermeté résiduelle pour le témoin A et le produit selon l'invention B en fonction du nombre de jours après récolte.

### EXEMPLE 1

Le complexe génique objet de la présente invention a été obtenu à partir de systèmes cellulaires constitués par du pollen de Cucumis melo.

Ces systèmes cellulaires ont été soumis à différents rayonnements suivant le protocole suivant :
1) Détermination de la dose de rayonnement après laquelle 50 % des cellules étaient encore vivantes. Ce rayonnement est communément appelé dose léthale 50 (DL 50).
2) Expositions des systèmes cellulaires à la DL 50.

Les systèmes cellulaires, ainsi irradiés ont été ensuite reproduits avec des systèmes cellulaires compatibles (issus de Cucumis mélo).

Les nouveaux systèmes cellulaires obtenus ont été cultivés puis criblés de façon à mettre en évidence la présente du complexe génique recherché. Le complexe a été criblé en utilisant la décroissance du taux d'acide propionique avec le temps. En effet, sur les cellules d'origine ce taux d'acide croît avec le temps.

Il est bien connu que l'éthylène (C₂H₄) est un des facteurs qui initialise, coordonne et accélère l'ensemble des processus physiologiques de la maturation des systèmes cellulaires climatériques.

Or, selon les études antérieures (De Pooter H.L., 1981 ; De Pooter H.L., 1982 ; De Pooter H.L., 1984) l'acide propionique interviendrait comme précurseur et régulateur de la synthèse de C₂H₄.

La décroissance du taux d'acide propionique en fonction du temps a donc été choisi comme confirmation de la création du complexe génique dans le système cellulaire.

Parallèlement, pour accélérer les processus de criblage, les mesures suivantes ont été effectuées :
1) Appréciation de la texture interne et du ramollissement du fruit en fonction du temps par mesure de la flexion totale sous un poids de 3 kg.
2) Pénétrométrie à maturation (indice réfractométrique supérieur à 11) pour mesurer la texture interne (norme AFNOR) avec élimination des mesures moyennes inférieures à 1,5 kgf.

Ainsi, on obtient des systèmes cellulaires non climatériques à partir de Cucumis melo qui peuvent être transférés par croisement, notamment dans des systèmes voisins compatibles ou bien dans d'autres systèmes par des méthodes de biologie moléculaire.

### EXEMPLE 2

Cet exemple a pour objet de comparer les caractéristiques d'un Cucumis melo modifié par un complexe génique selon l'invention (produit B) avec le Cucumis melo non modifié (produit A).

### a) Activité respiratoire et production de C₂H₄

C. melo est un fruit climatérique : lors de sa maturation, on peut observer une brusque augmentation de l'activité respiratoire et de l'émission de C₂H₄.

Les mesures d'activité respiratoire et d'émission de C₂H₄ sont effectuées à partir de la veraison jusqu'à la sénescence du fruit. Le C₂H₄ est dosé par chromatographie en phase gazeuse.

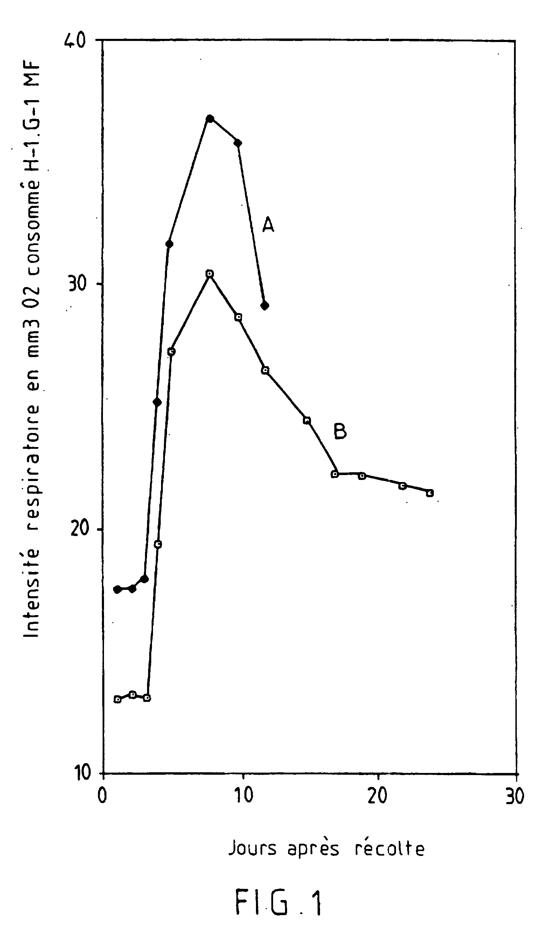

Les résultats représentés aux figures 1 et 2 correspondent à la moyenne de 6 échantillons.

C. melo selon l'invention a une activité respiratoire beaucoup moins importante que le témoin (figure 1).

Quelques jours après la récolte, comme cela est visible sur la figure 2, l'émission d'éthylène est plus faible chez le produit modifié. La quantité maximale d'éthylène dégagé est la même pour les deux produits. Cette émission maximale est observée 10 jours plus tard chez le produit de l'invention.

L'introduction du complexe génique transforme le pic éthylénique observé chez le témoin en un plateau stable pendant 8 jours pour le produit selon l'invention. On a donc une inactivation des gènes codant les enzymes impliqués dans la production d'éthylène autocatalytique.

Le complexe génique supprime la crise éthylénique du système cellulaire qui conserve alors ses fonctions physiologiques beaucoup plus longtemps. Le système cellulaire a réaction climatérique est transformé en un système cellulaire non climatérique.

A 20°C, la crise de maturation suivie de l'arrêt des fonctions physiologiques etducatabolisme du système cellulaire initial peut être caractérisée par l'arrêt de production de C₂H₄.

L'introduction du complexe génique permet une continuation des fonctions physiologiques pendant une quinzaine de jours à 20°C après la montée du dégagement de C₂H₄.

### b) Evolution des glucides

Les teneurs en sucres réducteurs et sucres totaux ont été étudiées au début et à la fin de la phase de maturité de consommation.

Cette phase s'étend sur 3 jours pour le produit A (les dosages ont été faits le premier et le troisième jour).

Chez le produit de l'invention (produit B), la durée de conservation est plus importante (les dosages sont effectués le premier et le septième jour).

Les sucres réducteurs sont principalement représentés par le fructose.

Une quantité de glucose relativement importante est présente chez le témoin à la fin de la période de maturité de consommation.

**Tableau I**

| Teneur en sucres des deux produits | | | |
|---|---|---|---|
| | | Sucres réducteurs g. 100 g⁻¹ MF | Sucres totaux g. 100 g⁻¹ MF |
| A | 1er jour | 0,95 | 5,6 |
| | 3ème jour | 1,5 | 6,4 |
| B | 1er jour | 0,15 | 8,0 |
| | 7ème jour | 0,65 | 7,8 |

Les résultats présentés dans le tableau I montrent que la teneur en sucres totaux est supérieure chez le produit de l'invention.

Il faut noter que la teneur en sucres réducteurs est très faible chez le produit de l'invention.

Le tableau II montre le comportement des systèmes cellulaires avant (A) et après (B) l'introduction du complexe génique.

L'introduction du complexe génique diminue considérablement la teneur en fructose à l'origine et permet une stabilité dans le temps de la teneur en glucose.

**Tableau II**

| | | Fructose g. 100 g⁻¹ MF | Glucose g. 100 g⁻¹ MF |
|---|---|---|---|
| A | 1er jour | 0,9 | 0,05 |
| | 3ème jour | 0,95 | 0,6 |
| B | 1er jour | 0,1 | 0,05 |
| | 7ème jour | 0,65 | 0,05 |

### c) Evolution des acides organiques

Les dosages des acides organiques par HPLC ont été effectués parallèlement aux dosages des sucres.

Le tableau III montre le comportement des systèmes cellulaires avant (A) et après (B) l'introduction du complexe génique.

L'introduction du complexe génique diminue considérablement la teneur initiale en acide malique et inverse le processus de l'acide propionique au cours du temps.

**Tableau III**

| | | Acide malique mg. 100 g⁻¹ MF | Acide propionique mg. 100 g⁻¹ MF |
|---|---|---|---|
| A | 1er jour | 18,5 | 190 |
| | 3ème jour | 11,1 | 370 |
| B | 1er jour | trace | 410 |
| | 7ème jour | 13,9 | 270 |

### d) Evolution des protéines

Le dosage des protéines, donc des acides aminés, a été fait par chromatographie liquide à haute performance par comparaisons avec des étalons de référence (Ex. : GABA, pure, ... et...).

Les résultats ci-dessous montrent que la teneur en acides aminés totaux, donc en protéines, est supérieure chez le produit de l'invention (B) :

Sur trois lots pour chaque espèce (A) témoin et quatre lots (B) melon selon l'invention, le dosage conduit à une quantité de 6,7 g de protéine pour 100 g de matière sèche dans le cas de A et 11,0 g de protéine pour 100 g de matière sèche pour B.

De plus, l'introduction du complexe génique augmente considérabelemnt le pourcentage d'Acide γ-aminobutyrique (GABA) par rapport aux autres acides aminés :
A : 3,3 %
B : 7,32 %

### e) Dosage de l'azote total

Le dosage de l'azote total a été fait sur la matière lyophilisée puis hydrolysée pendant 24 heures puis passée dans un analyseur automatique d'azote.

Le résultat montre que l'introduction du complexe génique augmente considérablement le pourcentage d'azote total par rapport à la matière sèche :

Sur cinq lots, on a obtenu une moyenne de 1,65 % pour A et 2,18 % pour B.

L'azote pur provenant des protéines, il est courant d'appliquer un coefficient multiplicateur sur ce pourcentage d'azote afin d'obtenir un taux de protéines. Ce résultat est en accord avec le paragraphe d) ci-dessus.

### f) Evolution des acides gras

Les teneurs en acides gras ont été mesurées à l'aide d'un chromatographe à colonnes en phase capillaire sur des échantillons dont les huiles ont été extraites à l'hexane sur le produit lyophilisé.

L'analyse des acides gras sous forme méthyl ester de différents types de melons montre que chez le produit de l'invention (produit B), il existe des acides gras en quantité non négligeable en dessous de C12, ce qui est très rare.

Le pourcentage d'acide palmitoléique (C16-1) est beaucoup plus important après l'introduction du complexe génique (produit B) par rapport au témoin.

Le pourcentage d'acide oléique (C18-1) diminue beaucoup après l'introduction du complexe génique.

### g) Etude de la texture

Les melons sont coupés en tranche de 1 cm d'épaisseur par une trancheuse ménagère.

Des rondelles de 2,5 cm de diamètre sont découpées à l'emporte-pièce sur les tranches de melon.

Ces rondelles sont réparties par lot de 20 dans 10 sachets et stockées à 10°C.

Cette expérience a été réalisée sur les melons avant et après introduction du complexe génique.

La mesure de la texture sera faite tous les jours (INSTRON TMSM). On mesure la pression de rupture de la structure des échantillons par compression entre deux plateaux.

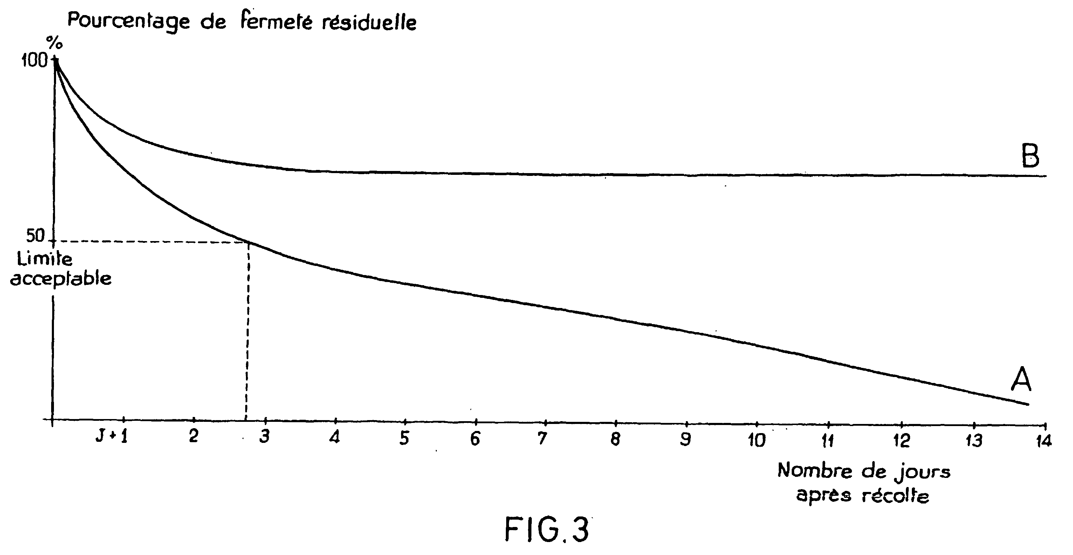

Les résultats sont indiqués en figure 3 (A avant introduction du complexe génique, B après introduction du complexe génique).

### Bibliographie

De Pooter H.L., Dirinck P.J., Willaert G.A., Schamp N.M., 1981, Phytochemistry 20, 9, 2135-2138.
De Pooter H.L., Montens J.P., Dirinck P.J., Willaert G.A., Schamp S.M., 1982, Phytochemistry 21, 5, 1015-1016.
De Pooter H.L., D'Y de walle Y.E., Willaert G.A., Dirinck P.J., Schamp N.M. 1984, Phytochemistry 23, 1, 23-26.

## Revendications

1. Cucumis melo modifié par un complexe génique obtenu à partir d'un système cellulaire végétal mutant du type Cucumis melo faisant s'exprimer dans ledit Cucumis melo modifié les phénotypes suivants, détectables au cours du temps et définis par comparaison avec le système cellulaire non modifié :
a) une émission d'éthylène présentant un plateau stable pendant environ 5 jours,
b) une diminution de la teneur en fructose,
c) une stabilité de la teneur en glucose dans le temps,
d) une diminution de la teneur en acide malique,
e) une augmentation sensible de la teneur en acide propionique dès l'introduction du complexe génique dans ledit système cellulaire, suivie d'une inversion de son processus d'évolution.

2. Cucumis melo selon la revendication 1, **caractérisé en ce que**:
- il présente un dégagement de C₂H₄ de l'ordre d'au moins 10 µl kg⁻¹.h⁻¹, 7 jours après le maximum de dégagement de C₂H₄,
- sa teneur en acide propionique diminue du premier au septième jour suivant la maturation.

3. Complexe génique obtenu a partir d'un système cellulaire végétal mutant du type Cucumis melo, **caractérisé en ce que**, lorsqu'il est introduit dans un Cucumis melo, il fait s'exprimer dans ledit système cellulaire les phénotypes suivants, détectables au cours du temps et définis par comparaison avec ledit système cellulaire non modifié:
a) une émission d'éthylène présentant un plateau stable pendant environ 5 jours,
b) une diminution de la teneur en fructose,
c) une stabilité de la teneur en glucose dans le temps,
d) une diminution de la teneur en acide malique,
e) une augmentation sensible de la teneur en acide propionique dès l'introduction du complexe génique dans ledit système cellulaire, suivie d'une inversion de son processus d'évolution.

4. Cellules végétables modifiées par l'introduction d'un complexe génique selon la revendication 3.

## Patentansprüche

1. Cucumis melo, modifiziert durch einen Genkomplex, der ausgehend von einem mutierten pflanzlichen zellulären System des Typs Cucumis melo erhalten wird und veranlasst, dass in dem Cucumis melo die folgenden Phänotypen ausgeprägt werden, die im Lauf der Zeit nachweisbar sind und durch Vergleich mit dem unmodifizierten zellulären System definiert sind:
a) eine Ethylen-Emission, die während etwa 5 Tagen ein stabiles Plateau aufweist,
b) eine Verringerung des Fructose-Gehalts,
c) eine Stabilität des Glucose-Gehalts über die Zeit,
d) eine Verringerung des Äpfelsäure-Gehalts,
e) eine deutliche Vermehrung des Propionsäure-Gehalts gleich nach Einführen des Genkomplexes in das zelluläre System, gefolgt von einer Umkehr seines Entwicklungsprozesses.

2. Cucumis melo nach Anspruch 1, **dadurch gekennzeichnet, dass**:
- er eine C₂H₄-Freisetzung in der Größenordnung von mindestens 10 µl kg⁻¹ · h⁻¹ 7 Tage nach der maximalen C₂H₄-Freisetzung aufweist,
- sein Gehalt an Propionsäure vom ersten bis zum siebten Tag nach der Reifung abnimmt.

3. Genkomplex, erhalten ausgehend von einem mutierten pflanzlichen zellulären System des Typs Cucumis melo, **dadurch gekennzeichnet, dass** er, wenn er in einen Cucumis melo eingeführt wird, veranlasst, dass in diesem zellulären System die folgenden Phänotypen ausgeprägt werden, die im Lauf der Zeit nachweisbar sind und durch Vergleich mit dem unmodifizierten zellulären System definiert sind:
a) eine Ethylen-Emission, die während etwa 5 Tagen ein stabiles Plateau aufweist,
b) eine Verringerung des Fructose-Gehalts,
c) eine Stabilität des Glucose-Gehalts über die Zeit,
d) eine Verringerung des Äpfelsäure-Gehalts,
e) eine deutliche Vermehrung des Propionsäure-Gehalts gleich nach Einführen des Genkomplexes in das zelluläre System, gefolgt von einer Umkehr seines Entwicklungsprozesses.

4. Pflanzliche Zellen, modifiziert durch die Einführung eines Genkomplexes nach Anspruch 3.

## Claims

1. Cucumis melo modified with a gene complex obtained from a Cucumis melo type mutant plant cell system which causes the following phenotypes, which are detectable over time and which are defined by comparison with the unmodified cell system, to be expressed in the said modified Cucumis melo:
a) an ethylene release which presents a stable plateau for about 5 days,
b) a decrease in the fructose content,
c) stability of the glucose content with time,
d) a decrease in the malic acid content,
e) a substantial increase in the propionic acid content on the introduction of the gene complex into the said cell system, followed by a reversal of its variation process.

2. Cucumis melo according to Claim 1, **characterized in that**:
- it exhibits a release of C₂H₄ of the order of not less than 10 µl kg⁻¹ h⁻¹, 7 days after the C₂H₄ release maximum,
- its propionic acid content decreases from the first to the seventh day, following maturation.

3. Gene complex obtained from a Cucumis melo type mutant plant cell system, **characterized in that**, when it is introduced into a Cucumis melo, it causes the following phenotypes, which are detectable over time and which are defined by comparison with the said unmodified cell system, to be expressed in the said cell system:
a) an ethylene release which presents a stable plateau for about 5 days,
b) a decrease in the fructose content,
c) stability of the glucose content with time,
d) a decrease in the malic acid content,
e) a substantial increase in the propionic acid content on the introduction of the gene complex into the said cell system, followed by a reversal of its variation process.

4. Plant cells modified by the introduction of a gene complex according to Claim 3.
